(19) Europäisches Patentamt
European Patent Office
Office européen des brevets

(11) **EP 2 463 380 A1**

(12) **EUROPÄISCHE PATENTANMELDUNG**

(43) Veröffentlichungstag:
**13.06.2012 Patentblatt 2012/24**

(51) Int Cl.:
*C12Q 1/58* *(2006.01)*     *G01N 33/543* *(2006.01)*

(21) Anmeldenummer: **10193920.5**

(22) Anmeldetag: **07.12.2010**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Cytonet GmbH & Co. KG**
**69469 Weinheim/Bergstrasse (DE)**

(72) Erfinder: **Aygen, Sitke, Dr.**
**51105 Köln (DE)**

(74) Vertreter: **Schrell, Andreas**
**Gleiss Grosse Schrell & Partner**
**Patentanwälte Rechtsanwälte**
**Leitzstrasse 45**
**70469 Stuttgart (DE)**

(54) **Verfahren zur Isolierung von Harnstoff unter Entfernung von störendem CO2**

(57)     Verfahren zur Isolierung von Harnstoff und Entfernung von $CO_2$ aus Plasmaproben umfassend folgende Schritte

a) Bereitstellen einer Plasmaprobe

b) Zugabe einer Säure, um $CO_2$ teilweise zu entfernen

c) Gefriertrocknen der Probe, um weiteres $CO_2$ zu entfernen und eine getrocknete Probe zu erhalten

d) Wiederauflösen der getrockneten Probe und Neutralisieren auf einen pH-Wert von 4 bis 6 mit einer Pufferlösung, wobei gegebenenfalls vor der Zugabe der Säure eine Filtration durchgeführt wird

Fig.1

EP 2 463 380 A1

**Beschreibung**

Die vorliegende Erfindung betrifft ein Verfahren zur Isolierung von Harnstoff in Blutproben.

Harnstoff ist eine organische Verbindung, die im menschlichen Organismus ein Endprodukt des Stoffwechsels von Stickstoffverbindungen darstellt. Beim Menschen wird Harnstoff mit dem Urin ausgeschieden.

Die Harnstoffbildung erfolgt überwiegend in den Leberzellen und teilweise in den Nieren. Bei der Harnstoffbildung im Körper gibt es verschiedene Erkrankungen, teilweise erblich, die erhebliche gesundheitliche Schäden bewirken können. Die Bestimmung der Harnstoffbildung ist ein Indikator für die Funktion der Leber, beispielsweise bei Lebertransplantationen oder der Transplantation von Leberzellen.

Tuchman et al., Pediatric Research 2008 (64), Seite 213 beschreiben beispielsweise einen Defekt der N-Acetylglutamat-Synthase und die Analyse der Harnstoffbildung.

Zur Messung der Harnstoffbildung erhielten Patienten oral $^{13}$C markiertes Natriumacetat verabreicht, das im Körper zur Bildung von $^{13}$C markiertem Harnstoff führte; aus $^{13}$C markiertem Acetat entsteht $^{13}$CO$_2$, das in $^{13}$C-Carbamoyl-Phosphat und anschließend in $^{13}$C Harnstoff überführt wird.

Die meisten chemischen Elemente existieren in der Natur als Gemische mehrerer stabiler oder radioaktiver Isotope. Isotopenhäufigkeiten werden normalerweise auch bei Tracerstudien mit angereicherten Verbindungen in der Einheit Atomprozent (atom-%) oder ppm angegeben. Zur Beschreibung der Variationen im Bereich natürlicher Häufigkeiten existiert die relative Delta-Skala in Promille (‰). Die δ-Werte (z.B. $\delta^{13}$C, $\delta^{15}$N, $\delta^{18}$O) werden als Differenz des jeweiligen Isotopenverhältnisses R ([schweres Isotop]/[leichtes Isotop], z.B. R$^{13}$C=[$^{13}$C]/[$^{12}$C]) der Probe gegenüber einem Standard, relativ zu diesem Standard definiert.

Der $\delta^{13}$C-Wert berechnet sich z.B. nach:

$$\delta^{13}C \;=\; \frac{R_{Probe} - R_{Standard}}{R_{Standard}} \;\cdot\; 1000 \;=\; \left(\frac{R_{Probe}}{R_{Standard}} - 1\right)\;\cdot\; 1000$$

Der Standard für Kohlenstoff ist ein Kalk, PDB (Pee Dee Belemnite). Der durch den Einbau von CO$_2$ in der Photosynthese gebundene Kohlenstoff ist generell an $^{13}$C abgereichert. Die meisten Pflanzen reduzieren CO$_2$ zu Kohlenhydraten nach dem Calvin-Benson- oder C3-Weg. Dies führt dazu, dass die Biomasse von C3-Pflanzen (zu diesen gehören die Nutzpflanzen Reis, Kartoffel, Soja, Zuckerrübe und Getreide) $\delta^{13}$C-Werte im Bereich -24 bis -32‰ aufweisen. Andere Pflanzen fixieren CO$_2$ nach dem Hatch-Slack- oder C4-Weg. Die $\delta^{13}$C-Werte von Produkten aus C4-Pflanzen (Mais, Hirse, Zuckerrohr) haben $\delta^{13}$C-Werte im Bereich -10 bis -16‰. Deswegen können $\delta^{13}$C-Werte zur Überprüfung der Herkunft und Originalität von organischen Substanzen verwendet werden.

Die Bestimmung des $\delta^{13}$C-Wertes von Plasma-Harnstoff erfolgt üblicherweise durch eine Umsetzung des Harnstoffs in CO$_2$ mit Hilfe eines Enzyms. Deshalb ist es wichtig, dass die aus Plasma isolierte Harnstoff-Lösung frei von fremdem CO$_2$ ist. Allerdings ist in der Regel immer CO$_2$ im Plasma vorhanden, entweder als gelöstes freies CO$_2$ oder als gebundenes CO$_2$ in der Form von Bikarbonat. Eine vollständige Befreiung des Plasma von CO$_2$ ist nicht einfach; zusätzlich muss verhindert werden, bei der Isolierung CO$_2$ in die Probe einzutragen.

Zur Isolierung des Harnstoffes aus Blutplasma verwenden Tuchman et al. ein Verfahren mit folgenden Schritten:

Eine Plasmaprobe von 0,5 ml wurde mit 0,5 ml H$_2$O und 40 μl Perchlorsäure 60% versetzt und präzipitiertes Protein abgetrennt. Anschließend ruhte der Behälter 30 Minuten, um eine Freisetzung von CO$_2$ zu erlauben. Nach Überführen in ein neues Gefäß und Einstellen des pH in den Bereich 6 bis 7 mit 300 μl KOH 1 M wurde präzipitiertes Kaliumperchlorat abgetrennt. Mit Hilfe einer Ionenaustauschersäule wurde restliches Bicarbonat entfernt.

Die Säule wurde mit 1 ml HCl 10 mM gewaschen und das Eluat in einem Glasbehälter bei 80°C getrocknet. Die Probe ruhte über Nacht in einem verschlossenen Behälter, in dem ein Stück Gaze, das mit Natriumhydroxid getränkt war, mit eingeschlossen war, um Reste von CO$_2$ abzufangen.

Anschließend wurde der Behälter mit Helium gespült und das Gefäß mit einem Gummistopfen luftdicht verschlossen. Es wurden 400 μl Kaliumphosphatpuffer 0.5 M pH 6.0 mit 3 mg Urease-Enzym/400 μl durch den Gummistopfen injiziert. Nach einer Stunde erfolgte eine Zugabe von 100 μL Phosphorsäure 20%, um CO$_2$ freizusetzten und die Urease-Reaktion zu stoppen. Das freigesetzte $^{13}$CO$_2$ wurde mit Hilfe eines isotope ratio mass spectrometer (IRMS) vermessen.

Die Untersuchungen der Anmelderin haben ergeben, dass das oben beschriebene Verfahren sehr empfindlich ist. Mehrere Quellen von Fremd-CO$_2$ können bei diesem Verfahren die gemessenen delta-Werte des aus Harnstoff

entstandenen $CO_2$ verfälschen. Es ist darüber hinaus auch sehr aufwändig und langwierig. Wegen der niedrigen Ausbeute an Harnstoff ist ein größeres Volumen (0,5 ml) von Plasma notwendig. Dies kann bei Kindern Probleme hervorrufen. Auch bei einer Crossvalidierung mit USA und Europa haben sich nicht nachvollziehbare Differenzen in den Ergebnissen gezeigt.

Dass bei dem Tuchman et al. beschriebenen Verfahren zur Harnstoffisolierung aus Plasma die Entfernung von störendem $CO_2$ nicht vollständig gelingt, kann man durch den Vergleich der $\delta^{13}C$-Werte von aus Harnstoff erzeugtem $CO_2$ nachweisen. Die von Tuchmann et al. beobachteten $\delta^{13}C$-Werte sind sehr niedrig -25 bis -26‰.

Der natürliche Harnstoff in Plasma hat einen $\delta^{13}C$-Wert von -19 bis -23‰, abhängig von der Ernährung.

Aufgabe der Erfindung war es, ein Verfahren bereitzustellen, das zumindest einige der Nachteile des bekannten Verfahrens überwindet.

Gelöst wird die Aufgabe durch ein Verfahren zur Isolierung von Harnstoff und Entfernung von $CO_2$ in Plasmaproben, umfassend folgende Schritte:

a) Bereitstellen einer Plasmaprobe enthaltend Harnstoff

b) Zugabe einer Säure, um $CO_2$ teilweise zu entfernen

c) Gefriertrocknen der Probe, um $CO_2$ zu entfernen und eine getrocknete Probe zu erhalten

d) Wiederauflösen der getrockneten Probe und Neutralisieren auf einen pH-Wert von 4 bis 6 mit einer Pufferlösung

Ausgangspunkt für die Isolierung des Harnstoffs in einer Plasmaprobe ist eine Plasmaprobe, die Harnstoff enthält. Plasmaproben können in bekannter Weise aus Blutproben gewonnen werden. Auf Grund der hohen Reproduzierbarkeit des erfindungsgemäßen Verfahrens genügen Plasmaproben mit einem Volumen im Bereich von 0,2 bis 0,3 ml, es können aber auch größere Mengen eingesetzt werden.

Die Plasmaprobe enthält zumindest Harnstoff mit einem natürlichen Isotopenverhältnis. Sie kann zusätzlich mit $^{13}C$ angereichertem Harnstoff versetzt sein. Sie kann aber auch durch Verabreichung von $^{13}C$ markierten Vorstufen, beispielsweise Acetat oder Bicarbonat, mit $^{13}C$ angereichert sein. Hiervon können verträgliche Salze, z.B. Na oder K-Salze verabreicht werden.

Erfindungsgemäß wird der Harnstoff gemessen durch Umsetzung des Harnstoffs mit Urease zur Freisetzung von $CO_2$, daher muss zunächst vorhandenes $CO_2$ entfernt werden.

In einer Ausführungsform der Erfindung wird zunächst eine Filtration durchgeführt. Hierzu wird zur Fällung Lösungsmittel, beispielsweise Acetonitril und/oder Ameisensäure zugesetzt. Durch diese Filtration können Proteine und Lipide vom Plasma abgetrennt werden. Besonders geeignet hierfür sind sogenannte HybridSPE$^{TM}$ Säulen, die von der Firma SUPELCO erhältlich sind. Diese eignen sich insbesondere auch zu Entfernung von Phospholipiden.

Es hat sich jedoch gezeigt, dass auf den Schritt der Abtrennung der Proteine und Lipide aus dem Plasma auch verzichtet werden kann und trotzdem sehr gut reproduzierbare Werte erhalten werden. Der Verzicht auf den Filtrationsschritt spart zum einen Zeit, zum anderen aber auch Kosten, die für entsprechende Filter anfallen.

Erfindungsgemäß wird eine Säure zugesetzt. Durch den Zusatz an Säure wird ein Teil des $CO_2$ aus der Plasmaprobe entfernt. Geeignet ist beispielsweise Phosphorsäure in einer Konzentration von etwa 20%. Bezogen auf eine Plasmaprobe von 0,3 ml genügt die Säure (z.B. Phosphorsäure) in einer Menge von etwa 50 $\mu$l. Selbstverständlich können auch andere Säuren eingesetzt werden.

Ein wesentlicher Schritt des erfindungsgemäßen Verfahrens ist die anschließende Gefriertrocknung der Probe. Gefriertrocknen ist ein Verfahren, bei dem eine Probe eingefroren wird und im Vakuum das enthaltende Wasser sublimiert. Erfindungsgemäß eignet sich dieses Verfahren hervorragend zur Entfernung von Restmengen $CO_2$ in der Probe.

Die erhaltene Probe wird anschließend wieder aufgelöst und auf einen pH-Wert im Bereich von 4 bis 6, vorzugsweise 5 bis 6 eingestellt. Zur Einstellung eignen sich insbesondere Pufferlösungen, beispielsweise Phosphatpufferlösungen mit einem pH von 9,0. Auch andere Pufferlösungen können eingesetzt werden. Die in Tuchman et al. eingesetzte Kalilauge ist besonders ungünstig, da sie teilweise größere Mengen $CO_2$ enthält.

In einer Ausführungsform der Erfindung wird nach dem Wiederauflösen der getrockneten Proben wird diese entgast, um Reste von Fremd-$CO_2$, insbesondere aus der Zugabe des Puffers auszutreiben. Geeignet hat sich das Anlegen eines Vakuums im Bereich von 1 bis 10 mbar für 2 bis 3 Stunden.

Das Ergebnis dies Verfahrens ist eine Probe, die den Harnstoff aus dem Plasma noch enthält, aber im wesentlichen frei ist von $CO_2$ aus dem Plasma.

Zur Bestimmung des Isotopenverhältnisses des Harnstoffs wird nun auf ein an sich bekanntes Verfahren die Umwandlung mit Urease, zurückgegriffen:

Die wieder aufgelöste Probe wird mit einem Schutzgas gespült, beispielsweise Helium, Stickstoff oder Argon und gasdicht verschlossen. Anschließend erfolgt eine Zugabe von Urease, um aus dem vorhandenen Harnstoff $CO_2$ zu bilden. Geeignet ist beispielsweise eine Urease, wie sie von der Firma Sigma erhältlich ist. Eine Menge von 20 bis 100 Einheiten Urease für eine Plasmaprobe von 0,3 ml hat sich als besonders geeignet erwiesen.

Anschließend erfolgt eine Inkubation der Lösung. Die Inkubation bei einer Temperatur von etwa 36°C für einen Zeitraum von etwa 60 Minuten hat sich als geeignet erwiesen.

Danach wird eine Säure zugesetzt, um die weitere Ureasereaktion zu stoppen. Zusätzlich wird durch die Zugabe der Säure das gebildete $CO_2$ freigesetzt. Im Hinblick auf die 0,3 ml große Plasmaprobe hat sich die Verwendung von 100 $\mu$l Phosphorsäure 20%ig als geeignet erwiesen. In dem gasdichten Behälter ist nun $CO_2$ freigesetzt worden, das aus der Umsetzung des Harnstoffs im Plasma stammt. Nun kann das Isotopenverhältnis des $CO_2$ bestimmt werden. Für diese Bestimmung eignet sich insbesondere IRMS. Bei der IRMS wird das Verhältnis zwischen $^{13}C$ und $^{12}C$ relativ zu einem Standard gemessen, wie oben ausgeführt.

Zur Prüfung der Durchführung kann es sinnvoll sein, vor der Zugabe von Urease zu prüfen, ob die Lösung frei von $CO_2$ ist. Dies kann beispielsweise mittels IR-MS Spektroskopie erfolgen.

In einer Anwendung der Erfindung werden zur Messung der Kinetik der Harnstoffbildung $^{13}C$ markierte Substanzen dem Patienten verabreicht, um die Bildung von $^{13}C$ markiertem Harnstoff zu bestimmen. In einer bevorzugten Ausführungsform der Erfindung wird eine Kinetik dadurch bestimmt, dass zunächst eine Blutprobe entnommen wird, bevor ein Patient $^{13}C$ markierte Harnstoffvorläufer einnimmt (Basalwert), gefolgt einer oder bevorzugt mehrere Blutprobennahme, nachdem der Patient $^{13}C$-markierte Harnstoffvorläufer eingenommen hat. Hierdurch kann die Bildung von $^{13}C$ markiertem Harnstoff überprüft werden.

Erfindungsgemäß können Plasmamengen von etwa 100 bis 200 oder 200 bis 300 ml eingesetzt werden, d.h. im Vergleich zum Verfahren von Tuchman, genügt eine Blutprobe mit halber Größe. Insbesondere wenn eine Kinetik bestimmt wird und das Verfahren bei Kindern angewandt wird, ist es vorteilhaft, wenn die Mengen an genommenem Blut besonders klein sind. Gegenüber dem Verfahren von Tuchman et al. hat das erfindungsgemäße Verfahren eine bessere Reproduzierbarkeit und ist weniger aufwändig.

### Figurenbeschreibung

Figur 1 zeigt die Reproduzierbarkeit des Verfahrens gemäß Beispiel 3.

Figur 2 zeigt die Eichkurve des Spiking-Experiments gemäß Beispiel 4.

Figur 3 zeigt die Reproduzierbarkeit des Verfahrens gemäß Beispiel 5.

Figur 4 zeigt die Eichkurve des Spiking-Experiment gemäß Beispiel 6.

Figuren 5 und 6 zeigen die Ergebnisse der Messung der Harnstoffbildung gemäß Beispiel 7.

Das Verfahren wird durch die folgenden Beispiele näher erläutert.

### Beispiel 1: Harnstoffisolation mit Filtration

Aus einer Blutprobe wurde Plasma gewonnen. 300 $\mu$l Plasma wurden mit 200 $\mu$l deionisiertem Wasser verdünnt. Zu der Probe wurden 100 $\mu$l Acetonitril mit 1% Ameisensäure zugesetzt. In dem Gefäß bildete sich ein Niederschlag. Die Proben wurden über eine HybridSPE-Säule abfiltriert.

Das Filtrat wurde mit 50 $\mu$l Phosphorsäure 1 M versetzt, eingefroren und lyophilisiert. Die Probe wurde mit einer entgasten Phosphatpufferlösung 0,5 M pH 9 auf einen pH-Wert von 5,5 eingestellt. Der Probenbehälter (Vacutainer) wurde mit Heliumgas gespült. Anschließend erfolgte die Zugabe von 70 $\mu$l einer Lösung enthaltend 15 mg/ml Jack Bean Urease Typ III der Firma Sigma in Phosphatpuffer. Die Probe wurde für eine Stunde bei 36°C inkubiert. Anschließend wurden durch das Septum 60 $\mu$l Phosphorsäure 20%ig injiziert, um die Ureasereaktion zu stoppen und $CO_2$ freizusetzen. Aus dem freigesetzten $CO_2$ erfolgte die Bestimmung des Isotopenverhältnisses mittels IRMS.

### Beispiel 2: Harnstoffisolation ohne Filtration

Das Verfahren wurde wie in Beispiel 1 durchgeführt, allerdings wurde auf die Zugabe von Acetonitril und Ameisensäure sowie die Filtration verzichtet, d.h. die Plasmaprobe wurde nach Zugabe von Säure direkt lyophilisiert. Das weitere Verfahren wurde identisch durchgeführt.

### Beispiel 3: Reproduzierbarkeit des Verfahrens

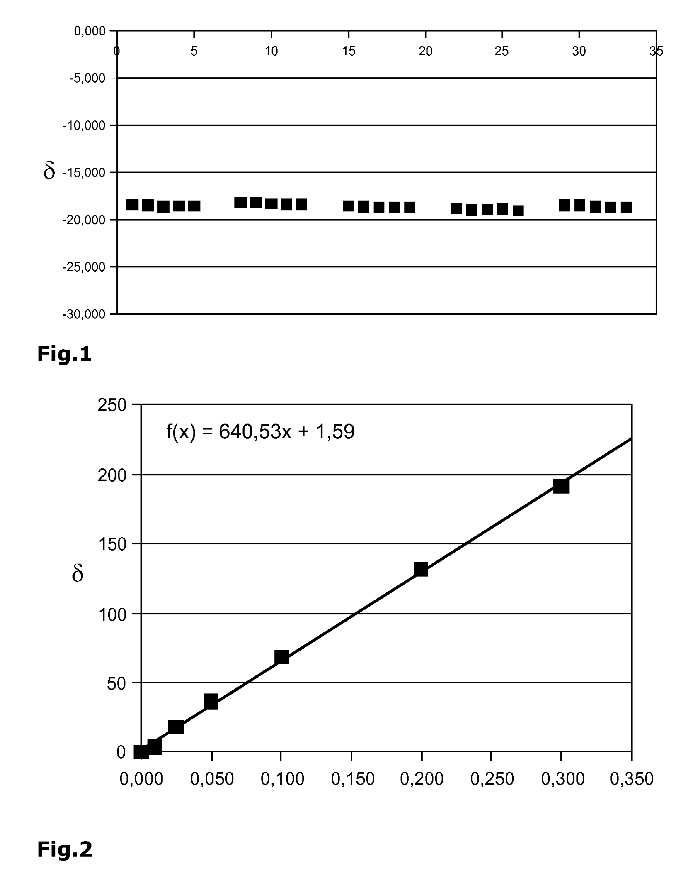

Eine Plasmaprobe wurde in fünf Proben unterteilt, die jeweils getrennt voneinander dem Verfahren gemäß Beispiel 1 unterzogen wurden. Jede erhaltene $CO_2$ Probe wurde fünfmal gemessen. Die Unterschiede sind minimal, vgl. Figur 1.

### Beispiel 4: Spiking-Experiment

Den Plasmaproben aus Beispiel 1 wurden 99 %ig markierter $^{13}$C Harnstoff in Mengen von 0,01 mg, 0,25 mg, 0,5 mg, 0,1 mg, 0,2 mg, 0,3 mg zugesetzt und die Probe gemäß Verfahren 1 behandelt. Figur 2 zeigt die entsprechenden Messwerte. Die Eichkurve liegt auf einer Geraden mit einem Korrelationskoeffizienten von 0,99923.

### Beispiel 5: Reproduzierbarkeit

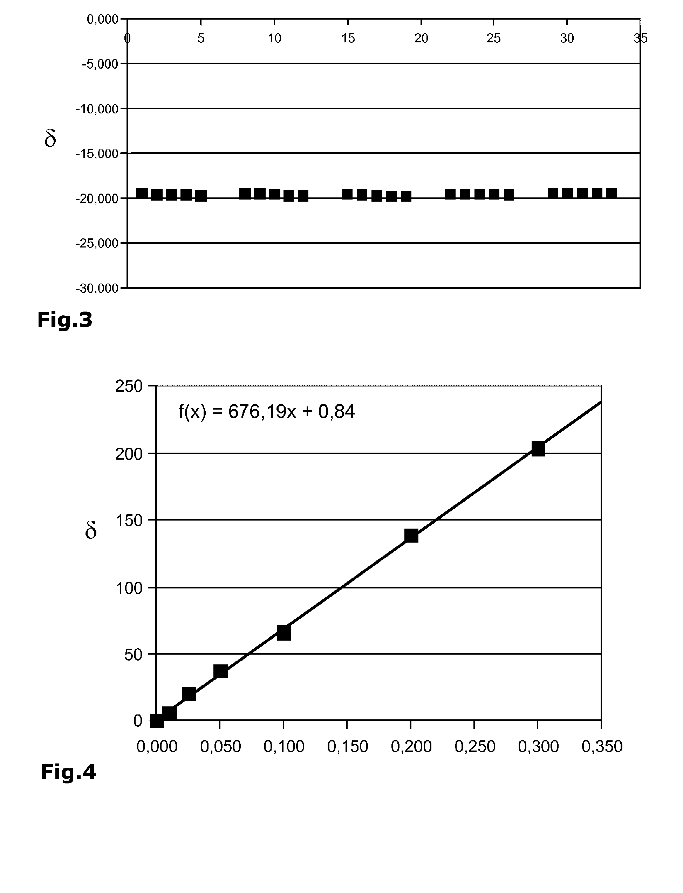

Die Messung der Reproduzierbarkeit gemäß Beispiel 3 wurde für das Verfahren ohne Filter gemäß Beispiel 2 wiederholt. Figur 3 zeigt die Ergebnisse. Auch hier besteht eine hervorragende Reproduzierbarkeit.

### Beispiel 6: Spiking-Experiment

Das Spiking-Experiment gemäß Beispiel 4 wurde wiederholt, wobei das Verfahren gemäß Beispiel 2 eingesetzt wurde. Die entsprechende Eichgerade ist in Figur 4 ersichtlich. Ihr Korrelationskoeffizient lag bei R=0,99959.

### Beispiel 7: Messung der Harnstoffbildung

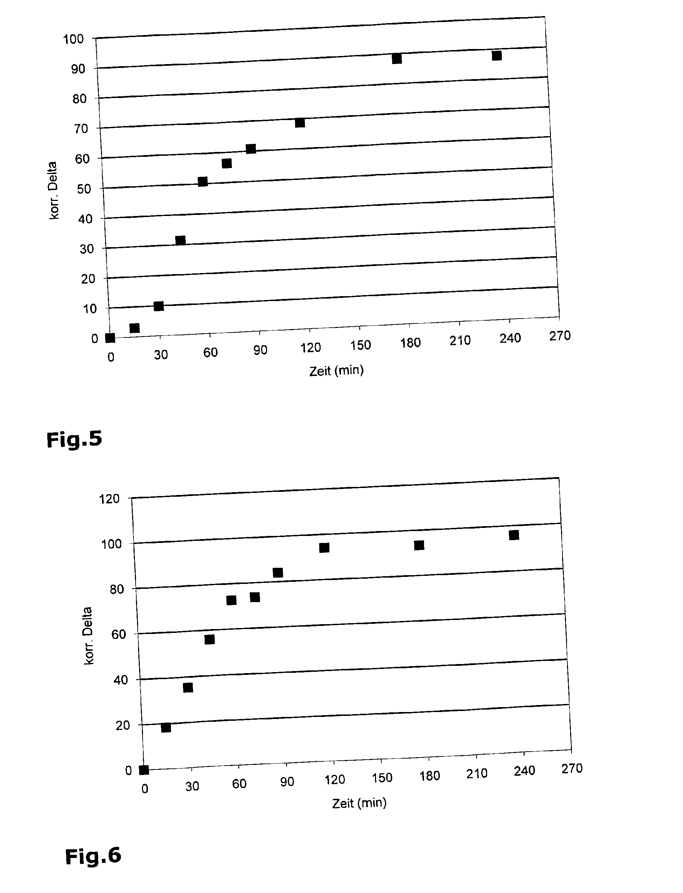

Zwei Probanden wurden Blut entnommen und Plasma hergestellt aus je **300** $\mu$l Plasma wurde gemäß dem erfindungsgemäßen Verfahren Harnstoff isoliert und das $^{13}$C/$^{12}$C- Isotopenverhältnis des Harnstoffs bestimmt.
Dabei wurde jede Probe 5-mal gemessen und über den Durchschnittwert der Basalwert ermittelt. Danach wurde den Probanden 27 mg/kg 99% $^{13}$C markiertes Na-Acetat verabreicht.
Die Prozedur der Blutentnahme und die Bestimmung des $^{13}$C/$^{12}$C- Isotopenverhältnisses des isolierten Harnstoffs wurde in 15, 30, 45, 60, 75, 90, 120, 180 und 240 Minuten Abstand wiederholt. Ein kleinerer Anteil des $^{13}$C markiertes Acetats wird im Körper in Harnstoff umgewandelt und ist aufgrund der Empfindlichkeit der Messung nachweisbar. Die Kinetik des neu gebildeten Harnstoffs wurde durch Messung der Delta Werte (Erhöhung der $^{13}$C/$^{12}$C-Isotopenverhältnisses) bestimmt. Die Kinetik der Harnstoffbildung ist in den Figuren 5 und 6 dargestellt.

## Patentansprüche

**1.** Verfahren zur Isolierung von Harnstoff und Entfernung von $CO_2$ aus Plasmaproben umfassend folgende Schritte

    a) Bereitstellen einer Plasmaprobe
    b) Zugabe einer Säure, um $CO_2$ teilweise zu entfernen
    c) Gefriertrocknen der Probe, um weiteres $CO_2$ zu entfernen und eine getrocknete Probe zu erhalten
    d) Wiederauflösen der getrockneten Probe und Neutralisieren auf einen pH-Wert von 4 bis 6 mit einer Pufferlösung.

**2.** Verfahren nach Anspruch 1, **dadurch gekennzeichnet, dass** vor der Zugabe einer Säure in Schritt b) eine Filtration durchgeführt wird.

**3.** Verfahren nach Anspruch 1 oder 2, **dadurch gekennzeichnet, dass** nach Schritt d) die Probe unter reduziertem Druck entgast wird.

**4.** Verfahren nach mindestens einem der Ansprüche 1 bis 3, **dadurch gekennzeichnet, dass** die Probe in Schritt d) auf einen pH-Wert von 5 bis 6 eingestellt wird.

**5.** Verfahren nach mindestens einem der Ansprüche 1 bis 4, **dadurch gekennzeichnet, dass** die Plasmaprobe von einem Probanden oder Patienten stammt, der vor der Entnahme $^{13}$C -markierte Harnstoffvorläufer eingenommen hat.

6. Verfahren zur Bestimmung des $^{13}$C-Isotopenverhältnisses von Harnstoff in einer Plasmaprobe umfassend die Schritte

   - Isolierung von Harnstoff durch ein Verfahren nach einem der Ansprüche 1 bis 5
   - Spülen mit Schutzgas
   - Zugabe von Urease, um $CO_2$ zu bilden
   - Inkubation
   - Zugabe einer Säure, um das gebildete $CO_2$ freizusetzen
   - Messung des $^{13}$C-Isotopenverhältnisses des freigesetzten $CO_2$.

7. Verfahren zur Diagnostik des Harnstoffstoffwechsels umfassend die Schritte

   - Bereitstellen einer ersten Plasmaprobe eines Patienten,
   - Bestimmung des $^{13}$C-Isotopenverhältnisses von Harnstoff in der ersten Plasmaprobe gemäß Anspruch 6,
   - Bereitstellen von mindestens einer weiteren Plasmaprobe, die von dem Patienten stammt, wobei der Patient vor der Entnahme $^{13}$C-markierte Harnstoffvorläufer eingenommen hat,
   - Bestimmung des $^{13}$C-Isotopenverhältnisses von Harnstoff in der mindestens einen weiteren Plasmaprobe gemäß Anspruch 6,
   - Quantifizierung der Menge des gebildeten Harnstoffs durch das $^{13}$C-Isotopenverhältnis des Harnstoffs in der ersten und der mindestens einen weiteren Plasmaprobe.

8. Verfahren nach Anspruch 7, **dadurch gekennzeichnet, dass** mindestens zwei weitere Plasmaproben verwendet werden.

9. Verfahren nach Anspruch 8, **dadurch gekennzeichnet, dass** die weiteren Plasmaproben im Zeitraum von 15 bis 240 min entnommen wurden, nachdem der Patient $^{13}$C-markierte Harnstoffvorläufer eingenommen hatte.

10. Verfahren nach einem der Ansprüche 8 oder 9, **dadurch gekennzeichnet, dass** die weiteren Plasmaproben im Abstand von 15 min entnommen wurden, nachdem der Patient $^{13}$C-markierte Harnstoffvorläufer eingenommen hatte.

**Fig.1**

f(x) = 640,53x + 1,59

**Fig.2**

**Fig.3**

f(x) = 676,19x + 0,84

**Fig.4**

**Fig.5**

**Fig.6**

Europäisches
Patentamt

European
Patent Office

Office européen
des brevets

**EUROPÄISCHER RECHERCHENBERICHT**

**Nummer der Anmeldung**

EP 10 19 3920

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | TUCHMAN MENDEL ET AL: "N-carbamylglutamate markedly enhances ureagenesis in N-acetylglutamate deficiency and propionic acidemia as measured by isotopic incorporation and blood biomarkers", PEDIATRIC RESEARCH, WILLIAMS AND WILKINS, BALTIMORE, MD, US, Bd. 64, Nr. 2, 1. August 2008 (2008-08-01) , Seiten 213-217, XP008127178, ISSN: 0031-3998, DOI: DOI:10.1203/PDR.0B013E318179454B * Seite 214 * ----- | 1-10 | INV. C12Q1/58 G01N33/543 |
| X | US 5 542 419 A (MOULTON-BARRETT REX [US] ET AL) 6. August 1996 (1996-08-06) * Spalte 1 - Spalte 2; Beispiele * ----- | 1-10 | |
| X | US 2008/090268 A1 (AYGEN SITKE [DE]) 17. April 2008 (2008-04-17) * Absatz [0013] - Absatz [0016] * ----- | 1-10 | |
| A | JP 55 069038 A (CHUGAI PHARMACEUTICAL CO LTD) 24. Mai 1980 (1980-05-24) * Zusammenfassung * ----- | 1-10 | RECHERCHIERTE SACHGEBIETE (IPC) C12Q G01N |
| X | EP 1 415 159 B1 (AYGEN SITKE [DE]) 24. Oktober 2007 (2007-10-24) * Spalte 2 - Spalte 4; Ansprüche * ----- | 1-10 | |
| | -/-- | | |

Der vorliegende Recherchenbericht wurde für alle Patentansprüche erstellt

| Recherchenort | Abschlußdatum der Recherche | Prüfer |
|---|---|---|
| München | 28. Juni 2011 | Gonçalves Mauger, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................................

& : Mitglied der gleichen Patentfamilie, übereinstimmendes Dokument

EPO FORM 1503 03.82 (P04C03)

Europäisches
Patentamt

European
Patent Office

Office européen
des brevets

# EUROPÄISCHER RECHERCHENBERICHT

Nummer der Anmeldung

EP 10 19 3920

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | CHEY W D ET AL: "The 13C-urea blood test accurately detects active Helicobacter pylori infection: a US, Multicenter trial", AMERICAN JOURNAL OF GASTROENTEROLOGY, ELSEVIER SCIENCE INC, US, Bd. 94, Nr. 6, 1. Januar 1999 (1999-01-01), Seiten 1522-1524, XP002245806, ISSN: 0002-9270, DOI: DOI:10.1111/J.1572-0241.1999.1137_R.X * Seite 1522 - Seite 1523 * ----- | 1-10 | |
| A | WO 2006/028648 A2 (JACKSON H M FOUND MILITARY MED [US]; BAKALTCHEVA IRINA [US]; WILDER DO) 16. März 2006 (2006-03-16) * Seite 12 - Seite 15 * ----- | 1-10 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| München | 28. Juni 2011 | Gonçalves Mauger, M |

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**
EP 10 19 3920

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-06-2011

| Im Recherchenbericht angeführtes Patentdokument | | Datum der Veröffentlichung | Mitglied(er) der Patentfamilie | | Datum der Veröffentlichung |
|---|---|---|---|---|---|
| US 5542419 | A | 06-08-1996 | KEINE | | |
| US 2008090268 | A1 | 17-04-2008 | KEINE | | |
| JP 55069038 | A | 24-05-1980 | JP | 1374735 C | 22-04-1987 |
| | | | JP | 61042826 B | 24-09-1986 |
| EP 1415159 | B1 | 24-10-2007 | AT | 376676 T | 15-11-2007 |
| | | | AU | 2002355464 A1 | 24-02-2003 |
| | | | BR | 0211803 A | 31-08-2004 |
| | | | CA | 2456546 A1 | 20-02-2003 |
| | | | CN | 1539082 A | 20-10-2004 |
| | | | DK | 1415159 T3 | 25-02-2008 |
| | | | WO | 03014744 A2 | 20-02-2003 |
| | | | EP | 1415159 A2 | 06-05-2004 |
| | | | ES | 2294150 T3 | 01-04-2008 |
| | | | MX | PA04001246 A | 06-06-2005 |
| | | | PT | 1415159 E | 26-11-2007 |
| WO 2006028648 | A2 | 16-03-2006 | US | 2008119818 A1 | 22-05-2008 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **TUCHMAN et al.** *Pediatric Research,* 2008, 213